# FASCICULE DE BREVET EUROPEEN

(11) **EP 2 986 856 B1**
(45) Date de publication et mention de la délivrance du brevet: **04.09.2019**
(21) Numéro de dépôt: 14717797.6
(22) Date de dépôt: 19.03.2014
(51) Int. Cl.: F04D 29/66, F04D 17/16, F04D 25/06, A61M 16/00

(54) **TURBINE POUR APPAREIL D'ASSISTANCE RESPIRATOIRE À ÉMISSIONS SONORES RÉDUITES**
TURBINE FÜR EINE ATEMHILFSVORRICHTUNG MIT VERMINDERTER GERÄUSCHEMISSION
TURBINE FOR A RESPIRATORY ASSISTANCE APPARATUS WITH REDUCED NOISE EMISSIONS

(30) Priorité: 18.04.2013 FR 1353512
(43) Date de publication de la demande: 24.02.2016
(73) Titulaire: Air Liquide Medical Systems, 92160 Antony (FR)
(72) Inventeur: DUBOIS, Pierre-Emmanuel, F-92330 Sceaux (FR)
(74) Mandataire: Pittis, Olivier
(86) Numéro de dépôt international: PCT/FR2014/050637
(87) Numéro de publication internationale: WO 2014/170571

(56) Documents cités:
- DE-B- 1 037 291
- FR-A1- 2 953 142
- JP-A- H0 894 146
- US-B1- 6 575 695

## Description

L'invention concerne une micro-soufflante ou turbine destinée à équiper un appareil d'assistance respiratoire. Plus particulièrement, l'invention porte sur l'utilisation des espaces morts à des fins acoustiques, par ajout de matériaux d'insonorisation adéquats dans lesdits espaces morts situés entre volute et pavillon.

Certains appareils d'assistance respiratoire mettent en oeuvre des micro-soufflantes ou turbines servant à générer un gaz sous pression, en général de l'air ou de l'air enrichi en oxygène, qui est ensuite envoyé vers les voies aériennes d'un patient.

Une turbine de ce type est notamment décrite par le document EP-A-2102504 qui enseigne un appareil d'assistance respiratoire permettant une délivrance régulée d'un gaz, notamment d'air, au moyen d'une turbine comprenant un boîtier, un conduit d'amenée d'air délimité par le boîtier, une volute dont l'ouverture d'entrée est en communication avec le conduit d'amenée d'air, une roue à ailettes, située immédiatement en aval de l'ouverture d'entrée de la volute, comprenant une ouverture d'entrée reliée à cette ouverture d'entrée de la volute et des orifices de sortie débouchant dans la volute, et un moteur d'entraînement de la roue en rotation de manière à générer un flux d'air centrifuge dans la volute.

Un des problèmes qui se pose avec les micro-soufflantes ou turbines est celui des émissions sonores, c'est-à-dire du bruit qu'elles génèrent. En effet, les appareils d'assistance respiratoire équipés de ces micro-soufflantes ou turbines sont destinés à être utilisés en milieu hospitalier ou à domicile, donc le bruit émis lors du fonctionnement du moteur et de la rotation de la roue à ailettes doit être aussi faible que possible.

Une autre turbine de ce type est décrite par le document FR 2 953 142...

Des solutions ont été proposées à cette fin, notamment une solution répandue consistant à insonoriser l'extérieur de la turbine avec un boitier d'insonorisation et/ou des mousses ou analogues disposées autour.

Ceci permet effectivement d'atténuer le bruit mais en engendrant des contraintes en termes d'encombrement, c'est-à-dire de volume occupé par les mousses.

Le problème qui se pose est d'obtenir une réduction du bruit émis par la turbine d'un appareil respiratoire dans un espace imposé, c'est-à-dire sans augmenter l'encombrement.

La solution est une turbine, encore appelée micro-soufflante, pour appareil d'assistance respiratoire comprenant :
- un moteur électrique coopérant avec une roue à ailettes pour générer un flux de gaz,
- un pavillon d'entrée comprenant une ouverture d'entrée par laquelle est aspiré du gaz, tel de l'air, lorsque la roue à ailettes est entraînée en rotation par le moteur électrique, la roue à ailettes étant située immédiatement en aval de l'ouverture d'entrée,
- une volute comprenant une partie inférieure de volute et une partie supérieure de volute, venant se raccorder l'une à l'autre et conformées de manière à former un compartiment interne englobant la roue à ailettes et au moins une partie d'un conduit d'évacuation servant à l'acheminement du gaz débité par ladite roue à ailettes, et
- le pavillon d'entrée étant agencé sur la partie supérieure de volute en définissant entre eux au moins un volume mort,
caractérisée en ce qu'au moins un volume mort comprend au moins un matériau d'insonorisation apte à atténuer au moins une partie des émissions sonores générées par le moteur et/ou la roue à ailettes lors d'une rotation de la roue à ailettes, le matériau d'insonorisation étant de type élastomère.

Selon le cas, la turbine ou micro-soufflante de l'invention peut comprendre l'une ou plusieurs des caractéristiques techniques suivantes :
- le moteur permet d'entrainer la roue à ailettes à une vitesse comprise entre 0 et 100 000 tr/min, typiquement entre 10 000 et 60 000 tr/min.
- la partie inférieure de volute et la partie supérieure de volute sont maintenues l'une à l'autre de manière rigide.
- une âme acoustique est enserrée entre le pavillon et la partie supérieure de volute.
- le pavillon est en élastomère.
- des moyens d'étanchéité permettent d'assurer une étanchéité fluidique entre la partie inférieure de volute et la paroi externe du moteur ou d'une enceinte contenant le moteur.
- la partie inférieure de volute et la partie supérieure de volute sont en matériau polymère, en particulier en polycarbonate ou ABS.

L'invention porte par ailleurs sur un appareil d'assistance respiratoire comprenant une turbine selon l'invention, en particulier il est choisi parmi les appareils de type CPAP (à pression continue ou un niveau de pression) ou BiPAP (à deux niveaux de pression).

La présente invention va maintenant être décrite plus en détail en références aux Figures annexées parmi lesquelles :
- la Figure 1 représente une vue schématique (en coupe) d'un mode de réalisation d'une micro-soufflante ou turbine pour appareil d'assistance respiratoire selon la présente invention, et
- la Figure 2 montre un matériau insonorisant agencé entre la volute et le pavillon de la turbine de la Figure 1.

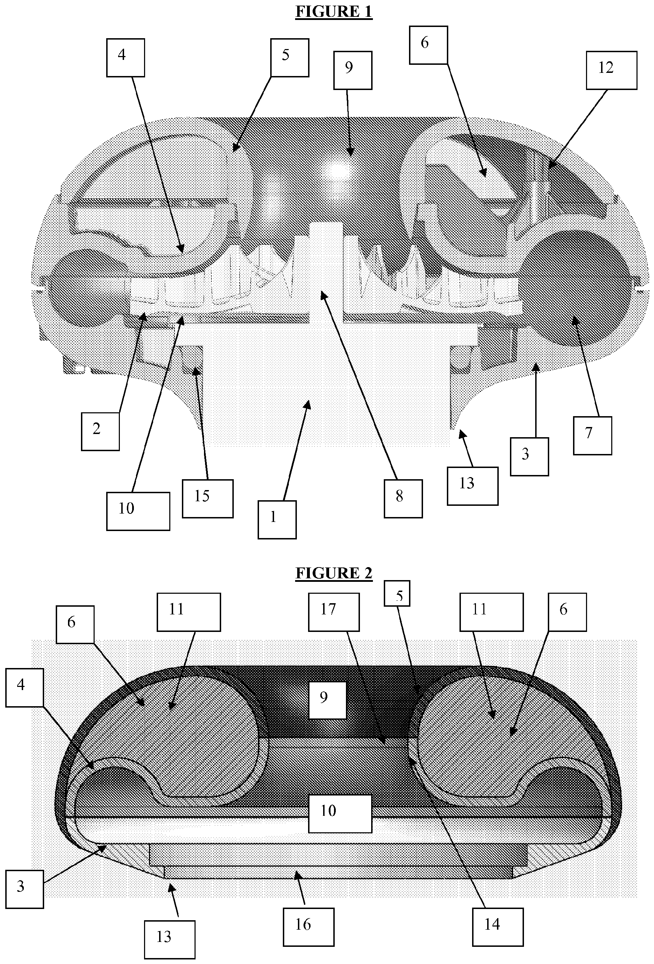

La Figure 1 schématise un mode de réalisation d'une micro-soufflante ou turbine selon l'invention équipant un appareil d'assistance respiratoire comprenant un moteur électrique 1 entrainant, via son axe rotatif 8, une roue à ailettes 2 servant à générer un flux de gaz, typiquement un flux d'air.

L'air est d'abord aspiré par la roue à ailettes 2 au travers du pavillon d'entrée 5 comprenant un passage central 9, c'est-à-dire une ouverture par laquelle transite l'air aspiré par la roue à ailettes 2 qui est située immédiatement en aval de l'ouverture d'entrée de la volute.

L'air est évacué ensuite par la roue à ailettes 2, via un conduit d'évacuation 7 de gaz, sous forme d'un flux d'air centrifuge engendré par la rotation de la roue à ailettes 2 lorsqu'elle est entraînée par le moteur 1.

Le conduit d'évacuation 7 est aménagé au sein d'une structure tridimensionnelle 3, 4 appelée « volute » comprenant une partie inférieure de volute 3, couramment appelée volute inférieure, et une partie supérieure de volute 4, appelée volute supérieure, venant se raccorder l'une à l'autre.

Les parties inférieure et supérieure de volute 3, 4 définissent entre elles un espace interne ou compartiment interne 10 englobant la roue à ailettes 2, c'est-à-dire que la roue à ailettes 2 est prise en sandwich dans le compartiment 10 formé entre les parties inférieure et supérieure de volute 3, 4.

Par ailleurs, les parties inférieure et supérieure de volute 3, 4 sont également conformées pour définir au moins une partie d'un conduit d'évacuation 7 servant à l'acheminement du gaz débité par ladite roue à ailettes 2.

La structure formant le pavillon d'entrée 5 vient se positionner sur la partie supérieure 4 de la volute 3, 4, c'est-à-dire que le pavillon d'entrée 5 surmonte la partie supérieure 4 de la volute 3.

En général, des espaces internes vides ou volumes morts 6 sont situés entre la paroi de la partie supérieure 4 de volute, c'est-à-dire la volute supérieure 4, et la paroi du pavillon d'entrée 5.

En d'autres termes, l'espace ou le (ou les) volume(s) 6 situé entre la volute 3, 4 et le pavillon 5, qui sont des structures de formes tridimensionnelles, est laissé vide, et constitue(nt) un ou des volumes morts.

Conformément à la présente invention, on utilise ces volumes morts 6 afin de résoudre le problème d'émissions sonores, c'est-à-dire pour atténuer le bruit généré par la turbine, lors de son fonctionnement.

Pour ce faire, comme illustré en Figure 2, conformément à la présente invention, les émissions sonores sont atténuées grâce à l'insertion dans tout ou partie desdits volumes morts 6 situés entre le pavillon 5 et la partie supérieure de volute 4, d'un (ou plusieurs) matériau insonorisant 11, encore appelé « âme acoustique ».

L'ajout d'une âme acoustique 11 dans l'espace mort 6 permet de stopper et/ou d'absorber au plus près, les émissions sonores générées par la rotation à grande vitesse de la roue 2 à ailettes, typiquement à des vitesses comprises entre 10 000 et 60 000 tours/min.

Un matériau insonorisant 11 susceptible d'être utilisé est un matériau souple soit de type élastomère, par exemple silicone, caoutchouc ou Thermo-Plastique-Elastomère, soit un métal tendre tel que le plomb, soit même une structure de mousse à cellules ouvertes ou fermées.

Par ailleurs, des moyens d'étanchéité 15, tel un joint ou analogue, permettent d'assurer une étanchéité fluidique entre la partie inférieure de volute 3 et la paroi externe du moteur 1 ou d'une enceinte contenant le moteur 1 de manière éviter des entrées ou des sorties d'air entre ces composants de la turbine.

Comme visible sur les Figures 1 et 2, les parties inférieure et supérieure de volute 3, 4 qui définissent entre elles le compartiment interne 10 dans lequel la roue à ailettes 2 est mise en rotation pour produire le flux de gaz, en particulier d'air, sont formées de deux structures tridimensionnelles venant se coupler l'une à l'autre.

Plus précisément, la partie inférieure de volute 3 a une forme générale de coupelle dont le fond 13 comporte une première ouverture 16 centrale (cf. Fig. 2) venant former manchon autour d'une partie de la paroi ou capotage externe du moteur (cf. Fig. 1) ou d'un compartiment renfermant le moteur 1, lorsque la partie inférieure de volute 3 est positionnée autour dudit moteur 1.

Par ailleurs, la partie supérieure de volute 4 forme une sorte de couvercle venant coiffer la partie inférieure de volute 3 comme illustré sur les Figures 1 et 2.

Elle comporte un étranglement 14 avec une seconde ouverture centrale 17 venant faire face à l'ouverture d'entrée 9 du pavillon d'entrée 5 de manière à ce que l'air aspiré par la roue 2, lors de sa rotation, transite successivement au travers de l'ouverture d'entrée 9 du pavillon puis de la seconde ouverture centrale 17.

Les profils de la paroi du pavillon 5 au niveau de l'ouverture d'entrée 9 et de la paroi de la partie supérieure de volute 4 au niveau de l'étranglement 14 sont arrondis de manière à améliorer la fluidité de la circulation de gaz, c'est-à-dire de l'air inspiré.

Le pavillon d'entrée 5 vient recouvrir de préférence la totalité de la partie supérieure de volute 4.

De préférence, le pavillon 5, l'axe 8 du moteur 1 et les parties inférieure et supérieure de volute 3, 4 sont agencées coaxialement les uns par rapport aux autres.

Typiquement, les parties inférieure et supérieure de volute 3, 4 sont en thermoplastique, alors que le pavillon 5 est préférentiellement en élastomère.

Le gaz, tel l'air, accéléré par la roue à ailettes 2 est récupéré par un conduit d'acheminement 7 qui permet de l'envoyer vers un circuit respiratoire relié à un patient.

Une turbine selon l'invention peut servir à traiter des pathologies respiratoires de tout type, en particulier apnée du sommeil, broncho-pneumopathie chronique obstructive ou BPCO, troubles liés à l'obésité...

Typiquement une turbine selon l'invention est aménagée dans un appareil d'assistance respiratoire de type CPAP (à pression continue) ou BiPAP (à deux niveaux de pression).

### Exemple comparatif

Afin de vérifier l'efficacité de la réduction des nuisances sonores grâce à une turbine selon l'invention, on a réalisé des tests comparatifs avec des turbines équipées ou non d'un matériau d'insonorisation dans les volumes morts situés entre le pavillon d'entrée et la partie supérieure de volute desdites turbines.

Le relevé du spectre acoustique est opéré à 1 mètre des turbines, latéralement, à la perpendiculaire de la sortie d'air (Mesure latérale ci-après), et par dessus, à la verticale de l'entrée d'air (Mesure verticale ci-après).

Le Tableau A donne la variation de bruit moyen avec une pondération A (LAeq) sur 1 minute par rapport à une turbine sans âme acoustique (turbine de référence hors invention) pour un régime de turbine de 1000 Hz.

**Tableau A**

| Turbine | Mesure latérale (dB A) | Mesure verticale (dB A) |
|---|---|---|
| Sans âme | Niveau sonore de référence | Niveau sonore de référence |
| Ame Silicone | -0,92 | -0,31 |
| Ame Plomb | -0,41 | 0,85 |

Le Tableau B est analogue au Tableau A, à caractéristiques identiques, mais exclusivement pour le bruit émis à la fréquence audible de 6300Hz.

**Tableau B**

| Turbine | Mesure latérale (dB A) | Mesure verticale (dB A) |
|---|---|---|
| Sans âme | Niveau sonore de référence | Niveau sonore de référence |
| Ame Silicone | -1,38 | -2,15 |
| Ame Plomb | -1,37 | -1,82 |

On constate que la solution de l'invention basée sur une incorporation d'un matériau d'insonorisation dans les volumes morts situés entre le pavillon d'entrée et la partie supérieure de volute permet d'atténuer efficacement les émissions sonores générées par le moteur et la roue à ailettes lors d'une rotation de la turbine.

Parmi les matériaux testés, le matériau d'insonorisation de type élastomère selon l'invention, à savoir une silicone, donne les meilleurs résultats en terme d'efficacité de contrôle des émissions acoustiques, par rapport au métal souple testé, à savoir ici du plomb.

On utilise donc un matériau d'insonorisation de type élastomère, notamment un thermo plastique élastomère ou TPE.

Plus généralement, la présente invention permet d'absorber les fréquences audibles dans la plage de fréquences allant de 20 à 20 000 Hz.

## Revendications

1. Turbine pour appareil d'assistance respiratoire comprenant :
- un moteur électrique (1) coopérant avec une roue à ailettes (2) pour générer un flux de gaz,
- un pavillon d'entrée (5) comprenant une ouverture d'entrée (9) par laquelle est aspiré du gaz lorsque la roue à ailettes (2) est entraînée en rotation par le moteur électrique (1), la roue à ailettes (2) étant située immédiatement en aval de l'ouverture d'entrée (9),
- une volute (3, 4) comprenant une partie inférieure de volute (3) et une partie supérieure de volute (4), venant se raccorder l'une à l'autre et conformées de manière à former un compartiment interne (10) englobant la roue à ailettes (2) et au moins une partie d'un conduit d'évacuation (7) servant à l'acheminement du gaz débité par ladite roue à ailettes (2), et
- le pavillon d'entrée (5) étant agencé sur la partie supérieure de volute (4) en définissant entre eux au moins un volume mort (6),
**caractérisée en ce que** le au moins un volume mort (6) comprend au moins un matériau d'insonorisation (11) apte à atténuer au moins une partie des émissions sonores générées par le moteur (1) et/ou la roue à ailettes (2) lors d'une rotation de la roue à ailettes (2) le matériau d'insonorisation (11) étant de type élastomère.

2. Turbine selon la revendication précédente, **caractérisée en ce que** le matériau d'insonorisation (11) est en un élastomère de type silicone, caoutchouc ou Thermo-Plastique-Elastomère.

3. Turbine selon l'une des revendications précédentes, **caractérisée en ce que** le moteur permet d'entrainer la roue à ailettes (2) à une vitesse comprise entre 0 et 100 000 tr/min, typiquement entre 10 000 et 60 000 tr/min.

4. Turbine selon l'une des revendications précédentes, **caractérisée en ce que** la partie inférieure de volute (3) et la partie supérieure de volute (4) sont maintenues l'une à l'autre de manière rigide.

5. Turbine selon l'une des revendications précédentes, **caractérisée en ce que** une âme acoustique est enserrée entre le pavillon (5) et la partie supérieure de volute (4).

6. Turbine selon l'une des revendications précédentes, **caractérisée en ce que** le pavillon (5) est en élastomère.

7. Turbine selon l'une des revendications précédentes, **caractérisée en ce que** des moyens d'étanchéité (15) permettent d'assurer une étanchéité fluidique entre la partie inférieure de volute (3) et la paroi externe du moteur (1) ou d'une enceinte contenant le moteur (1).

8. Turbine selon l'une des revendications précédentes, **caractérisée en ce que** la partie inférieure de volute (3) et la partie supérieure de volute (4) sont en matériau polymère, en particulier en polycarbonate ou ABS.

9. Appareil d'assistance respiratoire comprenant une turbine selon l'une des revendications précédentes.

10. Appareil d'assistance respiratoire selon la revendication 9, **caractérisé en ce qu'**il est choisi parmi les appareils de type CPAP ou BiPAP.

## Patentansprüche

1. Turbine für eine Atemhilfseinrichtung, umfassend:
- einen Elektromotor (1), der mit einem Flügelrad (2) zusammenwirkt, um eine Gasströmung zu erzeugen,
- einen Eingangspavillon (5), umfassend eine Eingangsöffnung (9), durch die Gas angesaugt wird, wenn das Flügelrad (2) vom Elektromotor (1) in Drehung versetzt wird, wobei sich das Flügelrad (2) unmittelbar stromabwärts der Eingangsöffnung (9) befindet,
- eine Spirale (3, 4), umfassend einen unteren Spiralenteil (3) und einen oberen Spiralenteil (4), die sich aneinander anschließen und angepasst sind, um ein inneres Fach (10) zu bilden, das das Flügelrad (2) und mindestens einen Teil einer Auslassleitung (7) umgibt, die der Durchleitung des vom Flügelrad (2) geförderten Gases dient, und
- wobei der Eingangspavillon (5) auf dem oberen Spiralenteil (4) angeordnet ist, indem er zwischen ihnen mindestens ein Totvolumen (6) definiert,
**dadurch gekennzeichnet, dass** das mindestens eine Totvolumen (6) mindestens ein Schalldämpfungsmaterial (11) umfasst, das geeignet ist, um mindestens einen Teil der vom Motor (1) und/oder dem Flügelrad (2) bei einer Drehung des Flügelrads (2) erzeugten Geräuschemissionen abzuschwächen, wobei das Schalldämpfungsmaterial (11) vom Typ Elastomer ist.

2. Turbine nach dem vorstehenden Anspruch, **dadurch gekennzeichnet, dass** das Schalldämpfungsmaterial (11) aus einem Elastomer vom Typ Silikon, Kautschuk oder thermoplastisches Elastomer ist.

3. Turbine nach einem der vorstehenden Ansprüche, **dadurch gekennzeichnet, dass** der Motor es erlaubt, das Flügelrad (2) bei einer Geschwindigkeit anzutreiben, die zwischen 0 und 100.000 U/min, typischerweise zwischen 10.000 und 60.000 U/min enthalten ist.

4. Turbine nach einem der vorstehenden Ansprüche, **dadurch gekennzeichnet, dass** der untere Spiralenteil (3) und der obere Spiralenteil (4) auf starre Weise aneinandergehalten werden.

5. Turbine nach einem der vorstehenden Ansprüche, **dadurch gekennzeichnet, dass** ein akustischer Steg zwischen dem Pavillon (5) und dem oberen Spiralenteil (4) eingespannt ist.

6. Turbine nach einem der vorstehenden Ansprüche, **dadurch gekennzeichnet, dass** der Pavillon (5) aus Elastomer ist.

7. Turbine nach einem der vorstehenden Ansprüche, **dadurch gekennzeichnet, dass** Abdichtmittel (15) es erlauben, eine fluidische Abdichtung zwischen dem unteren Spiralenteil (3) und der Außenwand des Motors (1) oder eines Raums, der den Motor (1) enthält, zu gewährleisten.

8. Turbine nach einem der vorstehenden Ansprüche, **dadurch gekennzeichnet, dass** der untere Spiralenteil (3) und der obere Spiralenteil (4) aus Polymermaterial, insbesondere aus Polycarbonat oder ABS sind.

9. Atemhilfseinrichtung, umfassend eine Turbine nach einem der vorstehenden Ansprüche.

10. Atemhilfseinrichtung nach Anspruch 9, **dadurch gekennzeichnet, dass** sie aus den Einrichtungen vom Typ CPAP oder BiPAP ausgewählt ist.

## Claims

1. Turbine for a respiratory assistance apparatus comprising:
- an electric motor (1) cooperating with a vaned rotor (2) for generating a gas stream,
- a bellmouth (5) comprising an inlet opening (9) through which gas is suctioned when the vaned rotor (2) is driven in rotation by the electric motor (1), with the vaned rotor (2) being located immediately downstream of the inlet opening (9),
- a volute (3, 4) comprising a lower portion of the volute (3) and an upper portion of the volute (4), connecting to one another and shaped so as to form an internal compartment (10) encompassing the vaned rotor (2) and at least one portion of a discharge conduit (7) used for the conveying of the gas delivered by said vaned rotor (2), and
- the bellmouth (5) being arranged on the upper portion of the volute (4) by defining between them at least one dead volume (6),
**characterised in that** the at least one dead volume (6) comprises at least one soundproofing material (11) capable of attenuating at least some of the noise emissions generated by the motor (1) and/or the vaned rotor (2) during a rotation of the vaned rotor (2) with the soundproofing material (11) being of the elastomer type.

2. Turbine according to the preceding claim, **characterised in that** the soundproofing material (11) is made of an elastomer of the silicone, rubber or Thermo-Plastic-Elastomer type.

3. Turbine according to one of the preceding claims, **characterised in that** the motor makes it possible to drive the vaned rotor (2) at a speed between 0 and 100,000rpm, typically between 10,000 and 60,000rpm.

4. Turbine according to one of the preceding claims, **characterised in that** the lower portion of the volute (3) and the upper portion of the volute (4) are rigidly held together.

5. Turbine according to one of the preceding claims, **characterised in that** a sound dampener is clamped between the bellmouth (5) and the upper portion of the volute (4).

6. Turbine according to one of the preceding claims, **characterised in that** the bellmouth (5) is made of elastomer.

7. Turbine according to one of the preceding claims, **characterised in that** sealing means (15) make it possible to ensure a fluidic seal between the lower portion of the volute (3) and the outer wall of the motor (1) or of an enclosure containing the motor (1).

8. Turbine according to one of the preceding claims, **characterised in that** the lower portion of the volute (3) and the upper portion of the volute (4) are made of a polymer material, in particular polycarbonate or ABS.

9. Respiratory assistance apparatus comprising a turbine according to one of the preceding claims.

10. Respiratory assistance apparatus according to claim 9, **characterised in that** it is selected from among the apparatuses of the CPAP or BiPAP type.
